# DEMANDE DE BREVET EUROPEEN

(11) **EP 1 138 318 A2**
(43) Date de publication de la demande: **04.10.2001**
(21) Numéro de dépôt: 01400746.2
(22) Date de dépôt: 22.03.2001
(51) Int. Cl.: A61K 7/13

(54) **Composition de teinture d'oxydation des fibres kératiniques et procédé de teinture mettant en oeuvre cette composition**

(30) Priorité: 30.03.2000 FR 0004061
(71) Demandeur: L'OREAL, 75008 Paris (FR)
(72) Inventeur: Plos, Grégory, 75011 Paris (FR)
(74) Mandataire: Fevrier, Murielle

(57) **Abrégé**

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins un colorant d'oxydation choisi parmi des pyridines de formule (I), et au moins un agent oxydant de nature enzymatique, ainsi que le procédé de teinture mettant en oeuvre cette composition.

## Description

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins un colorant d'oxydation choisi parmi des pyridines de formule (I), et au moins un agent oxydant de nature enzymatique, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que par exemple des indoles, des indolines ou bien encore des dérivés de pyridines, (voir notamment les demandes de brevet DE 3 132 885 et EP 0 106 987).

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes telles que la pyranose-oxydase, la glucose-oxydase ou bien l'uricase, en présence d'un donneur pour lesdites enzymes. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de leur intensité, (puissance), et de leur résistance vis à vis des diverses agressions que peuvent subir les cheveux.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant au moins un colorant d'oxydation choisi parmi les pyridines de formule (I) ci-après définie, et au moins un agent oxydant de nature enzymatique.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins un colorant d'oxydation choisi parmi les pyridines de formule (I) suivante et leurs sels d'addition avec un acide : 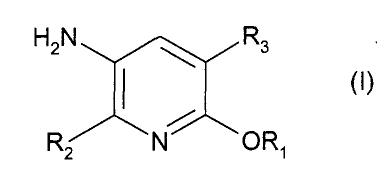 dans laquelle :

- R₁ représente un radical alkyle en C₁-C₄, monohydroxyalkyle en C₁-C₄, ou polyhydroxyalkyle en C₂-C₄,
- R₂ représente un radical alcoxy en C₁-C₄, , monohydroxyalcoxy en C₁-C₄, polyhydroxyalcoxy en C₂-C₄, amino, mono ou dialkyl(C₁-C₄)amino, monophénylamino, monohydroxyphénylamino, monoalcoxyphénylamino, mono ou dihydroxyalkyl(C₁-C₄)amino, mono ou di-(polyhydroxyalkyl(C₂-C₄)amino, alkyl(C₁-C₄)monohydroxyalkyle(C₁-C₄)amino, ou alkyl(C₁-C₄)polyhydroxyalkyle(C₂-C₄)amino,
- R₃ représente un atome d'hydrogène, un radical amino, monoalkyl(C₁-C₄)amino, monohydroxyalkyl(C₁-C₄)amino, ou mono(polyhydroxyalkyl(C₂-C₄)amino ;
étant entendu que lorsque R₂ représente un radical alcoxy en C₁-C₄, monohydroxyalcoxy en C₁-C₄ ou polyhydroxyalcoxy en C₂-C₄, alors R₃ représente un atome d'hydrogène ;
- et au moins un agent oxydant de nature enzymatique choisi parmi :
   a) un système comprenant les oxydo-réductases à 2 électrons et au moins un donneur pour lesdites oxydo-réductases à 2 électrons,
   b) les oxydo-réductases à 4 électrons,
   c) les peroxydases.

La composition tinctoriale prête à l'emploi conforme à l'invention conduit à des colorations particulièrement puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

Parmi les pyridines de formule (I) conforme à l'invention, on peut citer la 2,6-diméthoxy-3,5-diamino pyridine, la 2,3-diamino-6-méthoxy pyridine, la 2-méthylamino-3-amino-6-méthoxy pyridine, la 2-(2'-hydroxyphényl)amino-3-amino-6-méthoxy pyridine, la 2-(4'-hydroxyphényl)amino-3-amino-6-méthoxy pyridine, la 2-(4'-méthoxyphényl)amino-3-amino-6-méthoxy pyridine, la 2-phénylamino-3-amino-6-méthoxy pyridine, et leurs sels d'addition avec un acide.

Selon l'invention, la composition tinctoriale prête à l'emploi renferme de préférence de la 2,6-diméthoxy-3,5-diamino pyridine, et/ou au moins l'un de ses sels d'addition avec un acide.

La ou les pyridines de formule (I) utilisables à titre de colorant d'oxydation dans la composition tinctoriale prête à l'emploi conforme à l'invention représentent de préférence de 0,0001 à 10 % en poids environ du poids total de la composition tinctoriale, et encore plus préférentiellement de 0,005 à 5 % en poids environ de ce poids.

La composition tinctoriale prête à l'emploi conforme à l'invention peut contenir, en plus du ou des colorants d'oxydation de formule (I) ci-dessus, une ou plusieurs bases d'oxydation et/ou un ou plusieurs coupleurs.

Selon une forme de réalisation préférée de l'invention, la composition tinctoriale prête à l'emploi conforme à l'invention contient au moins une base d'oxydation.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (II) suivante et leurs sels d'addition avec un acide : 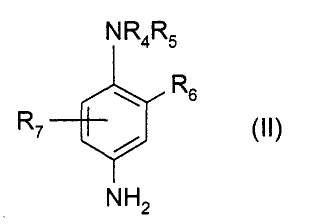 dans laquelle :
- R₄ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₆ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₇ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide : 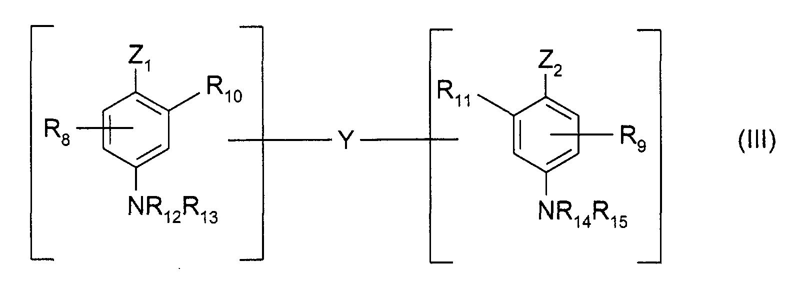 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₈ et R₉ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₀, R₁₁, R₁₂, R₁₃, R₁₄ et R₁₅, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (IV) suivante, et leurs sels d'addition avec un acide : 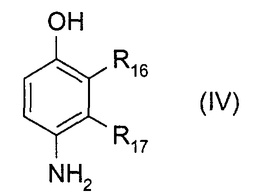 dans laquelle :
- R₁₆ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₇ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyràzolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon une forme de réalisation préférée de l'invention la ou les bases d'oxydation sont choisies parmi les paraphénylènediamines.

Lorsqu'elles sont présentes, la ou les bases d'oxydation représentent de préférence de 0,0005 à 12 % en poids environ du poids total de la composition tinctoriale prête à l'emploi conforme à l'invention, et encore plus préférentiellement de 0,005 à 8 % en poids environ de ce poids.

La ou les oxydo-réductases à 2 électrons, pouvant être utilisées à titre d'agent oxydant de nature enzymatique dans la composition tinctoriale prête à l'emploi conforme à l'invention, sont de préférence choisies parmi les pyranose oxydases, les glucose oxydases, les glycérol oxydases, les lactates oxydases, les pyruvate oxydases, et les uricases.

Selon l'invention, les oxydo-réductases à 2 électrons sont de préférence choisies parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent en général de 0,01 à 20 % en poids environ du poids total de la composition tinctoriale prête à l'emploi, de préférence de 0,1 à 10 % en poids environ de ce poids, et encore plus préférentiellement de 0,1 à 5 % en poids environ de ce poids.

On peut aussi définir la quantité d'oxydo-réductases à 2 électrons en fonction de son activité. Ainsi, l'activité enzymatique des oxydo-réductases à 2 électrons conformes à l'invention peut être définie à partir de l'oxydation du donneur en condition aérobie.

Une unité U correspond à la quantité d'oxydo-réductase à 2 électrons conduisant à la génération d'une µmole de peroxyde d'hydrogène par minute à un pH de 8,5 et à une température de 25°C.

De façon préférentielle, la quantité d'oxydo-réductase à 2 électrons est comprise entre 10 et 10⁸ unités U pour 100 g de composition tinctoriale prête à l'emploi conforme à l'invention.

Selon l'invention, on entend par donneur, les différents substrats participant au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; et enfin, à titre de donneur pour les bilirubine oxydases, on peut citer la bilirubine.

Lorsqu'ils sont utilisés, le ou les donneurs (ou substrats) représentent de préférence de 0,01 à 20 % en poids environ du poids total de la composition tinctoriale prête à l'emploi conforme à l'invention et encore plus préférentiellement de 0,1 à 5 % en environ de ce poids.

La ou les oxydo-réductases à 4 électrons, pouvant être utilisées à titre d'agent oxydant de nature enzymatique dans la composition tinctoriale prête à l'emploi conforme à l'invention, sont de préférence choisies parmi les laccases, les tyrosinases, les catéchol oxydases et les polyphénols oxydases.

Selon l'invention, et lorsqu'une oxydo-réductase à 4 électrons est utilisée, celle-ci est alors de préférence choisie parmi les laccases.

Ces laccases peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. Les laccases peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrats peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases utilisées selon l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

De manière générale, et lorsqu'elles sont utilisées, la ou les oxydo-réductases à 4 électrons représentent de préférence de 0,01 à 20 % en poids environ du poids total de la composition tinctoriale prête à l'emploi conforme à l'invention, et encore plus préférentiellement de 0,1 à 5 % en poids environ de ce poids.

De manière particulière, et lorsqu'une ou plusieurs laccases sont utilisées, la quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 2000 Lacu environ (soit entre 10000 et 40.10⁶ unités U environ ou soit entre 20 et 20.10⁶ unités ulac) pour 100 g de composition tinctoriale prête à l'emploi.

La ou les peroxydases, pouvant être utilisées à titre d'agent oxydant de nature enzymatique dans la composition tinctoriale prête à l'emploi conforme à l'invention, peuvent notamment être choisies parmi les enzymes appartenant à la sous-classe 1.11.1 décrite dans l'ouvrage Enzyme Nomenclature, Academic Press Inc., 1984. Certaines peroxydases appartenant à cette classe nécessitent la présence d'un donneur pour fonctionner. C'est le cas en particulier des NADH peroxydases (1.11.1) ayant pour donneur le NADH, des acide gras peroxydases (1.11.1.3) ayant pour donneur un acide gras tel que par exemple le palmitate, des NADPH peroxydases (1.11.1.2), ayant pour donneur le NADPH, des cytochrome-c peroxydases (1.11.1.5) ayant pour donneur le ferrocytochrome-c, des iodures peroxydases (1.11.1.8) ayant pour donneur les iodures, des chlorures peroxydases (1.11.10) ayant pour donneur les chlorures, des L. ascorbate peroxydases (1.11.1.11) ayant pour donneur le L. ascorbate, et des glutathion peroxydases (1.11.1.9) ayant pour donneur le glutathion.

D'autres peroxydases appartenant également à la sous-classe 1.11.1 fonctionnent sans donneur autre que le colorant d'oxydation ; il en est ainsi des catalases (1.11.1.6) et des peroxydases simplex (1.11.1.7).

Selon l'invention, on utilise de préférence les peroxydases simplex (1.11.1.7).

Toutes les peroxydases fonctionnent en présence de peroxyde d'hydrogène qui est apporté tel quel ou généré in situ par voie enzymatique à l'aide d'au moins une oxydo-réductase à 2 électrons et d'au moins un donneur pour ladite oxydo-réductase à 2 électrons, en présence d'air.

Les peroxydases pouvant être utilisées dans la composition tinctoriale prête à l'emploi conforme à l'invention peuvent être d'origine végétale, animale, fongique, bactérienne. Elles peuvent être également obtenues par biotechnologie.

Ainsi, les peroxydases peuvent par exemple être issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acétobacter peroxidans, Staphylococcus faecalis, ou encore Arthromyces ramosus.

L'unité d'activité de la peroxydase simplex (1.11.1.7) peut se définir comme étant la quantité d'enzyme simplex formant 1mg de purpurogalline à partir du pyrogallol en 20 secondes à un pH de 6 et à 20°C. A titre d'exemple, la peroxydase de radis noir P6782 de SIGMA ® présente une activité d'environ 250 unités par mg.

La concentration d'utilisation de ce type d'enzyme varie donc entre 25 et 5.10⁶ unités pour 100 g de composition prête à l'emploi conforme à l'invention.

Lorsqu'elles sont utilisées, la ou les peroxydases représentent de préférence de 0,0001 à 20% en poids environ du poids total de la composition prête à l'emploi conforme à l'invention, et encore plus préférentiellement de 0,001 à 10% en poids environ de ce poids.

Selon une forme de réalisation particulièrement préférée, la composition tinctoriale prête à l'emploi conforme à l'invention renferme au moins une oxydo-réductase à 4 électrons choisie parmi les laccases.

Les coupleurs pouvant être présents dans la composition prête à l'emploi conforme à l'invention sont choisis parmi les coupleurs classiquement utilisés en teinture d'oxydation et parmi les lesquels on peut citer les métaphénylènediamines, les méta-aminophénols, les métadiphénols, les naphtols et les coupleurs hétérocycliques tels que les pyrazolo-[1,5-b]-1,2,4-triazoles, les pyrazolo-[3,2-c]-1,2,4-triazoles, les pyrazol-5-ones, les pyridines différentes des pyridines de formule (I) ci-dessus, les indoles, les indolines, les indazoles, les benzimidazoles, les benzothiazoles, les benzoxazoles, les 1,3-benzodioxoles et les quinolines.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole, et leurs sels d'addition avec un acide.

Lorsqu'ils sont présents, le ou les coupleurs représentent de préférence de 0,0001 à 10 % en poids environ du poids total de la composition tinctoriale prête à l'emploi conforme à l'invention et encore plus préférentiellement de 0,005 à 5 % en poids environ de ce poids.

La composition tinctoriale prête à l'emploi conforme à l'invention peut également contenir un ou plusieurs colorants directs notamment pour modifier les nuances ou les enrichir en reflets.

D'une manière générale, les sels d'addition avec un acide utilisables dans le cadre des compositions tinctoriales de l'invention (pyridines de formule (I), bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de l'enzyme utilisée soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (VI) suivante : 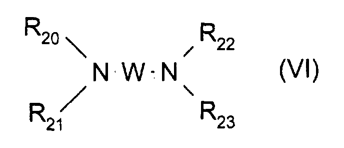 dans laquelle W est un reste propylène éventuellement substitué par un groupement hydroxyle ou un radical alkyle en C₁-C₄ ; R₂₀, R₂₁, R₂₂ et R₂₃, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et le ou les agents oxydants de nature enzymatique sont présents au sein de la même composition prête à l'emploi, et par conséquent ladite composition doit être exempte d'oxygène gazeux, de manière à éviter toute oxydation prématurée des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation choisi parmi les pyridines de formule (I) telle que définie précédemment, et d'autre part, une composition (B) renfermant, dans un milieu approprié pour la teinture, au moins un agent oxydant de nature enzymatique choisi parmi a) les oxydo-réductases à 2 électrons en présence d'au moins un donneur pour lesdites oxydo-réductases à 2 électrons, b) les oxydo-réductases à 4 électrons, c) les peroxydases, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES 1 à 9 DE TEINTURE

On a préparé les compositions tinctoriales prêtes à l'emploi suivantes, (teneurs en grammes) :

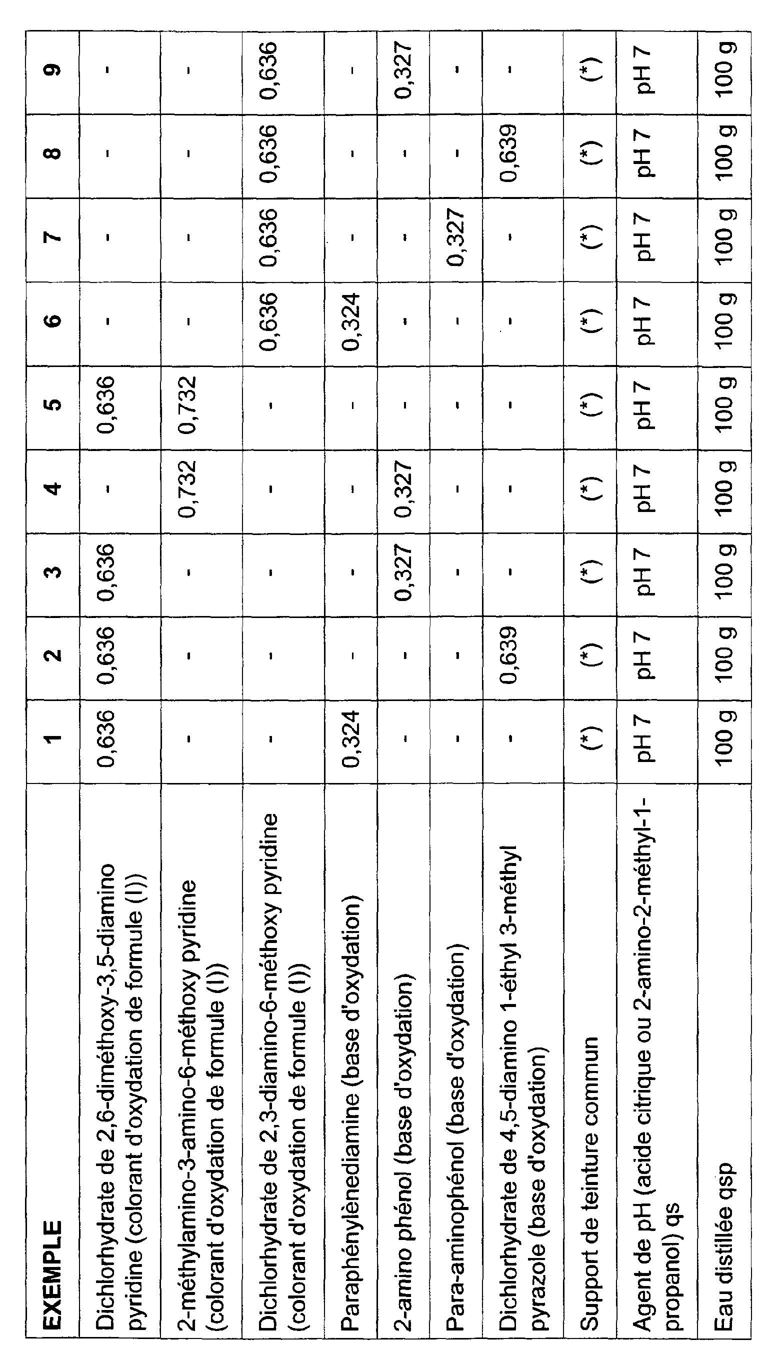

### (*) Support de teinture commun :

- Carboxyméthylchitosane à 84 % de matière active (M.A.) dans l'eau vendu sous la dénomination OLEVASAN ® par la société Sino-Lion 2,0 g
- Chlorure d'hydroxypropyl guar triméthylammonium vendu sous la dénomination Jaguar C13S ® par la société Rhodia Chimie 2,0 g
- Monooléate de polyglycérol (10 moles) vendu sous la dénomination DECAGLYN 10 ® par la société Nikko 1,0 g
- N-acétyl-L-cystéine 0,05 g
- Laccase 5,0 g

La quantité de laccase utilisée dans le support de teinture correspond à 5 360 000 unités ulac pour 100 g de composition tinctoriale prête à l'emploi.

Les compositions tinctoriales prêtes à l'emploi décrites ci-dessus ont été appliquées sur des mèches de cheveux gris naturels à 90 % de blancs pendant 30 minutes à température ambiante. Les cheveux ont ensuite été rincés, lavés avec un shampooing standard, puis séchés.

Les cheveux ont été teints dans les nuances figurant dans le tableau ci-après :

| **EXEMPLE** | **NUANCE OBTENUE** |
|---|---|
| **1** | Bleu noir |
| **2** | Acajou rouge |
| **3** | Vert bronze |
| **4** | Doré intense légèrement mat |
| **5** | Vert doré |
| **6** | Violet noir |
| **7** | Vert bronze doré cendré |
| **8** | Vert bleuté intense |
| **9** | Doré vert bronze |

### EXEMPLE 10 DE TEINTURE

On a préparé la composition prête à l'emploi suivante :
- Dichlorhydrate de 2,6-diméthoxy-3,5-diamino pyridine (colorant d'oxydation de formule (I)) 0,726 g
- Paraphénylènediamine (base d'oxydation) 0,324 g
- Acide urique 1,0 g
- Uricase 1,0 g
- Terpolymère acide méthacrylique / acrylate d'éthyle / méthacrylate de stéaryle oxyéthyléné vendu sous la dénomination Aculyn 22 ® par la société Rohm et Haas 2,5 g
- Monooléate polyglycérolé (10 moles) vendu sous la dénomination DECAGLYN 10 ® par la société Nikko 1,0 g
- N-acétyl-L-cystéine 0,1 g
- 2-amino-2-méthyl-1-propanol qs pH = 9,5
- Eau distillée qsp 100 g

La quantité d'uricase utilisée dans cette composition prête à l'emploi correspond à 20 000 unités U pour 100 g de composition prête à l'emploi.

La composition tinctoriale prête à l'emploi décrite ci-dessus a été appliquée sur des mèches de cheveux gris naturels à 90 % de blancs pendant 30 minutes à température ambiante. Les cheveux ont ensuite été rincés, lavés avec un shampooing, rincés à nouveau puis séchés.

Les cheveux ont été teints dans une nuance bleu cendré intense.

## Revendications

1. Composition prête à l'emploi, pour la teinture d'oxydation des fibres kératiniques et en particulier des fibres kératiniques humaines telles que les cheveux, **caractérisée par** le fait qu'elle comprend, dans un milieu approprié pour la teinture :
- au moins un colorant d'oxydation choisi parmi les pyridines de formule (I) suivante et leurs sels d'addition avec un acide : 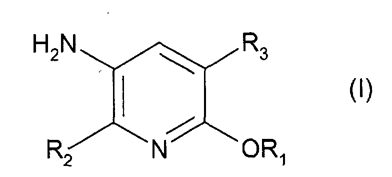 dans laquelle :
- R₁ représente un radical alkyle en C₁-C₄, monohydroxyalkyle en C₁-C₄, ou polyhydroxyalkyle en C₂-C₄,
- R₂ représente un radical alcoxy en C₁-C₄, , monohydroxyalcoxy en C₁-C₄, polyhydroxyalcoxy en C₂-C₄, amino, mono ou dialkyl(C₁-C₄)amino, monophénylamino, monohydroxyphénylamino, monoalcoxyphénylamino, mono ou dihydroxyalkyl(C₁-C₄)amino, mono ou di-(polyhydroxyalkyl(C₂-C₄)amino, alkyl(C₁-C₄)monohydroxyalkyle(C₁-C₄)amino, ou alkyl(C₁-C₄)polyhydroxyalkyle(C₂-C₄)amino,
- R₃ représente un atome d'hydrogène, un radical amino, monoalkyl(C₁-C₄)amino, monohydroxyalkyl(C₁-C₄)amino, ou mono(polyhydroxyalkyl(C₂-C₄)amino ;
étant entendu que lorsque R₂ représente un radical alcoxy en C₁-C₄, monohydroxyalcoxy en C₁-C₄ ou polyhydroxyalcoxy en C₂-C₄, alors R₃ représente un atome d'hydrogène ;
- et au moins un agent oxydant de nature enzymatique choisi parmi :
a) un système comprenant les oxydo-réductases à 2 électrons et au moins un donneur pour lesdites oxydo-réductases à 2 électrons,
b) les oxydo-réductases à 4 électrons,
c) les peroxydases.

2. Composition selon la revendication 1, **caractérisée par** le fait que les pyridines de formule (I) sont choisies parmi la 2,6-diméthoxy-3,5-diamino pyridine, la 2,3-diamino-6-méthoxy pyridine, la 2-méthylamino-3-amino-6-méthoxy pyridine, la 2-(2'-hydroxyphényl)amino-3-amino-6-méthoxy pyridine, la 2-(4'-hydroxyphényl)amino-3-amino-6-méthoxy pyridine, la 2-(4'-méthoxy-phényl)amino-3-amino-6-méthoxy pyridine, la 2-phénylamino-3-amino-6-méthoxy pyridine, et leurs sels d'addition avec un acide.

3. Composition selon la revendication 2, **caractérisée par** le fait qu'elle contient de la 2,6-diméthoxy-3,5-diamino pyridine, et/ou au moins l'un de ses sels d'addition avec un acide.

4. Composition selon l'une quelconque des revendications précédentes, **caractérisée par** le fait que la ou les pyridines de formule (I) représentent de 0,0001 à 10 % en poids du poids total de la composition tinctoriale.

5. Composition selon la revendication 4, **caractérisée par** le fait que la ou les pyridines de formule (I) représentent de 0,005 à 5 % en poids du poids total de la composition tinctoriale.

6. Composition selon l'une quelconque des revendications précédentes, **caractérisée par** le fait qu'elle renferme au moins une base d'oxydation choisie parmi les paraphénylènediamines, les bases doubles, les para-aminophénols, les ortho aminophénols et les bases d'oxydation hétérocycliques.

7. Composition selon la revendication 6, **caractérisée par** le fait que les paraphénylènediamines sont choisies parmi les composés de formule (II) suivante et leurs sels d'addition avec un acide : 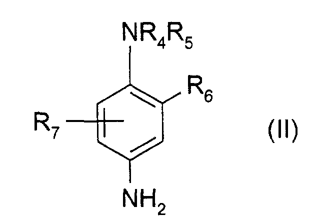 dans laquelle :
- R₄ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₆ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₇ représente un atome d'hydrogène, d'halogène ou un radical alkyle en C₁-C₄.

8. Composition selon la revendication 7, **caractérisée par** le fait que les paraphénylènediamines de formule (II) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

9. Composition selon la revendication 6, **caractérisée par** le fait que les bases doubles sont choisies parmi les composés répondant à la formule (III) suivante, et leurs sels d'addition avec un acide : 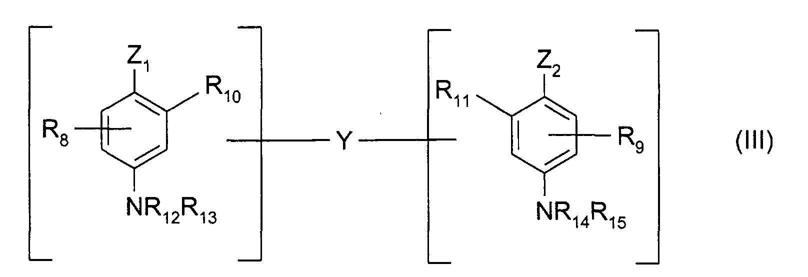 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₈ et R₉ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₀, R₁₁, R₁₂, R₁₃, R₁₄ et R₁₅, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

10. Composition selon la revendication 9, **caractérisée par** le fait que les bases doubles de formules (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

11. Composition selon la revendication 6, **caractérisée par** le fait que les para-aminophénols sont choisis parmi les composés répondant à la formule (IV) suivante, et leurs sels d'addition avec un acide : 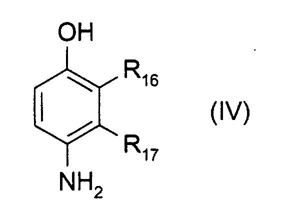 dans laquelle :
- R₁₆ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₇ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

12. Composition selon la revendication 11, **caractérisée par** le fait que les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

13. Composition selon la revendication 6, **caractérisée par** le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

14. Composition selon la revendication 6, **caractérisée par** le fait que les bases hétérocycliques sont choisies parmi les dérivés pyridiniques, les dérivés pyrimidiniques, les dérivés pyrazoliques, et leurs sels d'addition avec un acide.

15. Composition selon l'une quelconque des revendications 6 à 14, **caractérisée par** le fait que la ou les bases d'oxydation représentent de 0,0005 à 12 % en poids du poids total de la composition tinctoriale prête à l'emploi.

16. Composition selon l'une quelconque des revendications précédentes, **caractérisée par** le fait que la ou les oxydo-réductases à 2 électrons sont choisies parmi les pyranose oxydases, les glucose oxydases, les glycérol oxydases, les lactates oxydases, les pyruvate oxydases, et les uricases.

17. Composition selon la revendication 16, **caractérisée par** le fait que la ou les oxydo-réductases à 2 électrons sont choisies parmi les uricases d'origine animale, microbiologique ou biotechnologique.

18. Composition selon l'une quelconque des revendications 1 à 17, **caractérisée par** le fait que la ou les oxydo-réductases à 2 électrons représentent de 0,01 à 20 % en poids du poids total de la composition tinctoriale prête à l'emploi.

19. Composition selon l'une quelconque des revendications 1 à 18, **caractérisée par** le fait que le ou les donneurs représentent de 0,01 à 20 % en poids du poids total de la composition tinctoriale prête à l'emploi.

20. Composition selon l'une quelconque des revendications 1 à 15, **caractérisée par** le fait que la ou les oxydo-réductases à 4 électrons sont choisies parmi les laccases, les tyrosinases, les catéchol oxydases et les polyphénols oxydases.

21. Composition selon la revendication 20, **caractérisée par** le fait que la ou les oxydo-réductases à 4 électrons sont choisies parmi les laccases.

22. Composition selon l'une quelconque des revendications 1 à 15 ou 21, **caractérisée par** le fait que la ou les oxydo-réductases à 4 électrons représentent de 0,01 à 20 % en poids du poids total de la composition tinctoriale prête à l'emploi.

23. Composition selon l'une quelconque des revendications 1 à 15, **caractérisée par** le fait que la ou les peroxydases sont choisies parmi les NADH peroxydases ayant pour donneur le NADH, les acide gras peroxydases ayant pour donneur un acide gras, les NADPH peroxydases ayant pour donneur le NADPH, les cytochrome-c peroxydases ayant pour donneur le ferrocytochrome-c, les iodures peroxydases ayant pour donneur les iodures, les chlorures peroxydases ayant pour donneur les chlorures, les L. ascorbate peroxydases ayant pour donneur le L. ascorbate, et les glutathion peroxydases ayant pour donneur le glutathion, les catalases et les peroxydases simplex.

24. Composition selon la revendication 23, **caractérisée par** le fait que la ou les peroxydases sont choisies parmi les peroxydases simplex.

25. Composition selon la revendication 23 ou 24, **caractérisée par** le fait que la ou les peroxydases représentent de 0,0001 à 20% en poids du poids total de la composition prête à l'emploi.

26. Composition selon l'une quelconque des revendications précédentes, **caractérisée par** le fait qu'elle renferme au moins un coupleur choisi parmi les métaphénylènediamines, les méta-aminophénols, les métadiphénols, les naphtols et les coupleurs hétérocycliques tels que les pyrazolo-[1,5-b]-1,2,4-triazoles, les pyrazolo-[3,2-c]-1,2,4-triazoles, les pyrazol-5-ones, les pyridines différentes des pyridines de formule (I) telles que décrites à l'une quelconque des revendications 1 à 3, les indoles, les indolines, les indazoles, les benzimidazoles, les benzothiazoles, les benzoxazoles, les 1,3-benzodioxoles et les quinolines.

27. Composition selon la revendication 26, **caractérisée par** le fait que les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole, et leurs sels d'addition avec un acide.

28. Composition selon la revendication 26 ou 27, **caractérisée par** le fait que le ou les coupleurs représentent de 0,0001 à 10 % en poids du poids total de la composition tinctoriale prête à l'emploi.

29. Composition selon l'une quelconque des revendications précédentes, **caractérisée par** le fait que les sels d'addition avec un acide sont choisis parmi les chlorhydrates, les bromhydrates, les sulfates et les tartrates, les lactates et les acétates.

30. Procédé de teinture d'oxydation des fibres kératiniques et en particulier des fibres kératiniques humaines telles que les cheveux, **caractérisé par** le fait qu'on applique sur lesdites fibres, au moins une composition tinctoriale prête à l'emploi telle que définie dans l'une quelconque des revendications précédentes, pendant un temps suffisant pour développer la coloration désirée.

31. Dispositif de teinture à plusieurs compartiments, **caractérisé par** le fait qu'il comporte au moins un premier compartiment renfermant une composition (A) comprenant dans un milieu approprié pour la teinture, au moins un colorant d'oxydation choisi parmi les pyridines de formule (I) telle que définie à l'une quelconque des revendications 1 à 5 et, un deuxième compartiment renfermant une composition (B) comprenant, dans un milieu approprié pour la teinture, au moins un agent oxydant de nature enzymatique choisi parmi a) un système comprenant les oxydo-réductases à 2 électrons et au moins un donneur pour lesdites oxydo-réductases à 2 électrons, b) les oxydo-réductases à 4 électrons, c) les peroxydases.

32. Procédé de teinture d'oxydation des fibres kératiniques et en particulier des fibres kératiniques humaines telles que les cheveux, **caractérisé par** le fait qu'il comprend le mélange d'une composition (A) telle que définie à la revendication 31 avec une composition (B) telle que définie à la revendication 31, puis à procéder à l'application de ce mélange sur les fibres kératiniques.
